# EUROPEAN PATENT SPECIFICATION

(11) **EP 2 886 052 B1**
(45) Date of publication and mention of the grant of the patent: **18.07.2018**
(21) Application number: 13837210.7
(22) Date of filing: 17.09.2013
(51) Int. Cl.: A61B 5/055

(54) **MAGNETIC RESONANCE IMAGING DEVICE**
VORRICHTUNG ZUR ABBILDUNG MAGNETISCHER RESONANZEN
DISPOSITIF D'IMAGERIE PAR RÉSONANCE MAGNÉTIQUE

(30) Priority: 14.09.2012 US 201213618001
(43) Date of publication of application: 24.06.2015
(73) Proprietor: Toshiba Medical Systems Corporation, Otawara-shi, Tochigi-ken 324-8550 (JP)
(72) Inventor: ZHOU, Xiangzhi, Vernon Hills, Illinois 60061 (US); MIYAZAKI, Mitsue, Vernon Hills, Illinois 60061 (US); HOSHINO, Tsutomu, Vernon Hills, Illinois 60061 (US)
(74) Representative: Moreland, David
(86) International application number: PCT/JP2013/075056
(87) International publication number: WO 2014/042275

(56) References cited:
- WO-A1-2005/044104
- JP-A- 2005 137 558
- JP-A- 2005 510 322
- JP-A- 2006 087 626
- JP-A- 2006 087 626
- JP-A- 2009 535 139
- JP-A- 2011 083 592
- JP-A- 2012 105 982
- US-A- 5 377 681
- US-A1- 2006 241 412
- US-A1- 2009 005 672
- US-A1- 2009 220 050
- US-A1- 2011 071 382
- US-A1- 2011 249 005
- US-A1- 2012 078 085
- US-A1- 2012 082 352
- US-B1- 6 564 080

## Description

### Field

Embodiments relate to a magnetic resonance imaging apparatus.

### Background

Conventionally, there is a method referred to as myocardial perfusion as a method of capturing cardiac images by magnetic resonance imaging. Myocardial perfusion is a method of successively capturing T1-weighted images at high speed and thus observing, as changes over time, infusion of a gadolinium (Gd)-based contrast agent in myocardium to evaluate the blood supply to the myocardium.

The document US 2009/005672 A1 discloses a magnetic resonance imaging apparatus displaying myocadium perfusion images in bull's eye plots.

### Brief Description of Drawings

FIG. 1 is a high-level schematic block diagram of an MRI system according to an exemplary embodiment configured to provide non-contrast dynamic MRI myocardial perfusion analysis and visualization.
FIG. 2A is a first schematic depiction of a first exemplary tag-on, tag-off MRI data acquisition sequence for use in the MRI system according to the embodiment.
FIG. 2B is a schematic depiction of a second exemplary tag-on, tag-off MRI data acquisition sequence for use in the MRI system according to the embodiment.
FIG. 3 depicts a region to which tag-pulses are applied according to the embodiment.
FIG. 4 illustrates subtraction of tag-on and tag-off images on a pixel-by-pixel basis using complex-valued arithmetic so as to generate intensity images of the tag-on and tag-off images according to the embodiment.
FIG. 5A illustrates left ventricle intensity images at different BBTIs before registration according to the embodiment.
FIG. 5B illustrates left ventricle intensity images at different BBTIs after registration according to the embodiment.
FIG. 6A illustrates segmentation imposed on tag-on or tag-off images according to the embodiment.
FIG. 6B illustrates a region of interest to be composited into a subtracted image according to the embodiment.
FIG. 6C illustrates a region of interest to be composited into a clear image according to the embodiment where non-myocardial signals outside the segmented volume have been removed.
FIG. 7A depicts an exemplary perfusion visualization according to the embodiment wherein LV slice images before segmentation are displayed together in one display panel as a function of BBTI time periods.
FIG. 7B depicts an exemplary perfusion visualization according to the embodiment wherein LV slice images after segmentation are displayed together in one display panel as a function of BBTI time periods.
FIG. 7C depicts an exemplary perfusion visualization according to the embodiment wherein LV slice images after segmentation are displayed together in one display panel as a function of BBTI time periods.
FIG. 8A depicts coronary artery territory segmentation in the left ventricle according to the embodiment, here using an American Heart Association (AHA) six-segment model resulting in a color-mapped display of the segmented exemplary coronary artery territory of the left ventricle.
FIG. 8B depicts coronary artery territory segmentation in the left ventricle according to the embodiment, here using an AHA six-segment model resulting in a color-mapped display of the segmented exemplary coronary artery territory of the left ventricle.
FIG. 8C depicts coronary artery territory segmentation in the left ventricle according to the embodiment, here using an AHA six-segment model resulting in a color-mapped display of the segmented exemplary coronary artery territory of the left ventricle.
FIG. 8D depicts coronary artery territory segmentation in the left ventricle according to the embodiment, here using an American Heart Association (AHA) six-segment model resulting in a color-mapped display of the segmented exemplary coronary artery territory of the left ventricle.
FIG. 9A depicts a simultaneous one-panel display visualization of perfusion curves for each segment in each of different slices as a function of BBTI values using different exemplary visualization presentations according to the embodiment.
FIG. 9B depicts a simultaneous one-panel display visualization of perfusion curves for each segment in each of different slices as a function of BBTI values using different exemplary visualization presentations according to the embodiment.
FIG. 9C depicts a simultaneous one-panel display visualization of perfusion curves for each segment in each of different slices as a function of BBTI values using different exemplary visualization presentations according to the embodiment.
FIG. 10A illustrates a new type of bull's eye map for better visualization and understanding of relationships between all or any of BBTI, signal intensity (SI) which is related to perfusion, and LV slice numbers constituting substantially contiguous slices within a 3D image of the LV.
FIG. 10B illustrates a new type of bull's eye map for better visualization and understanding of relationships between all or any of BBTI, signal intensity which is related to perfusion, and LV slice numbers constituting substantially contiguous slices within a 3D image of the LV.
FIG. 11 is a schematic illustration of an exemplary computer program code structure for use in the MRI system of FIG. 1 (or a separate image processing apparatus) in the form of a flow chart for implementing exemplary embodiments of a system for analyzing BBTI tag-on and tag-off images acquired by magnetic resonance imaging.

### Description of Embodiments

A magnetic resonance imaging apparatus according to an embodiment is defined by independent claim 1. A magnetic resonance imaging apparatus (hereinafter, MRI (Magnetic Resonance Imaging) as appropriate) and an image processing apparatus according to an embodiment will be described below with reference to the drawings. Note that embodiments are not limited to the following. The contents of U.S. Patent Application No. 13/587,294 filed by the applicant on August 16th, 2012 and published as US 2014/0050379 is referred to hereby.

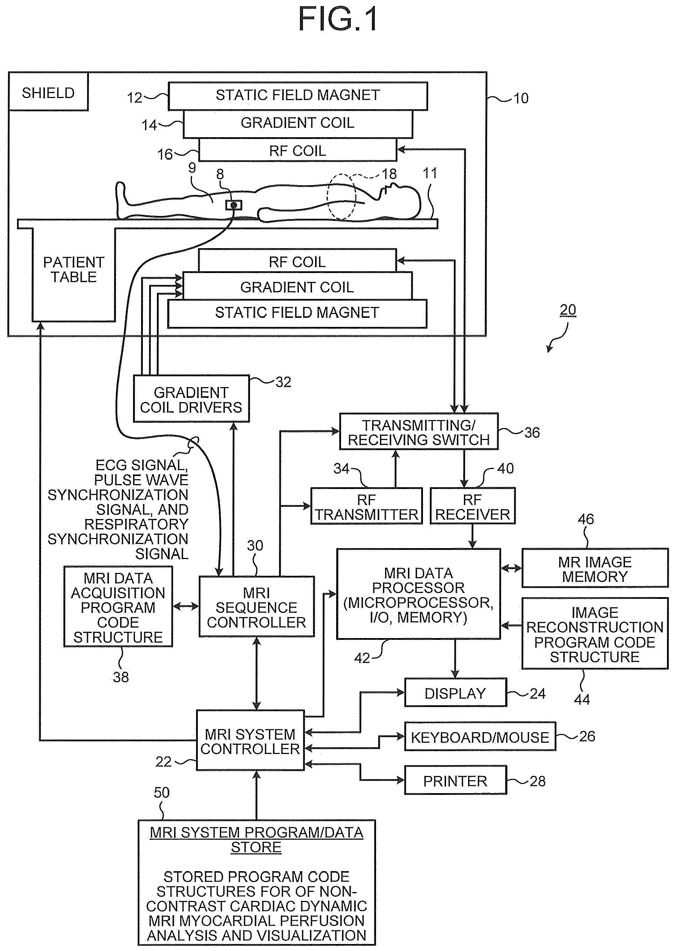

FIG. 1 is a high-level schematic block diagram of an MRI system according to an exemplary embodiment configured to provide non-contrast dynamic MRI myocardial perfusion analysis and visualization. The MRI system shown in FIG. 1 includes a gantry 10 (shown in schematic cross-section) and various related system components 20 interfaced therewith. At least the gantry 10 is typically located in a shielded room. The MRI system geometry depicted in FIG. 1 includes a substantially coaxial cylindrical arrangement of a static field Bₒ magnet 12, a Gx, Gy and Gz gradient coil set 14 and an RF (Radio Frequency) coil assembly 16. Along the horizontal axis of this cylindrical array of elements is an imaging region 18 shown as substantially encompassing the anatomical tissue of interest (i.e., region of interest (ROI) for a patient 9 (e.g., the heart for cardiac MRI) supported by a patient bed or table 11.

An MRI system controller 22 has input/output ports connected to a display 24, a keyboard/mouse 26 and a printer 28. As will be appreciated, the display 24 may be of the touch-screen variety so that it provides control inputs as well.

The MRI system controller 22 interfaces with an MRI sequence controller 30 which, in turn, controls Gx, Gy and Gz gradient coil drivers 32, as well as an RF transmitter 34 and a transmit/receive switch 36 (if the same RF coil is used for both transmission and reception). As those skilled in the art will appreciate, many different types of RF coils (e.g., whole body coils, surface coils, birdcage coils, coil arrays, etc.) may be employed to transmit and/or receive RF signals to/from the ROI in the imaging volume. As will also be appreciated, one or more suitable physiological transducers 8 may be affixed to the patient's body to provide all or any of gating signals of ECG (electrocardiogram) signal, respiratory synchronizing signal, and pulse wave synchronizing signal to the MRI sequence controller 30. The MRI sequence controller 30 also has access to a suitable program code structure 38 for executing MRI data acquisition sequences already available in the repertoire of the MRI sequence controller 30 - e.g., to generate non-contrast cardiac MRI tissue images using operator and/or system inputs defining particular MRI data acquisition sequence parameters, one or more ROI, etc.

The MRI system components 20 include an RF receiver 40 providing input to a data processor 42 so as to create processed image data which may be sent to the display 24. The MRI data processor 42 is also configured for access to an image reconstruction program code structure 44 and to an MR image memory 46 (e.g., for storing MR image data derived from processing in accordance with the exemplary embodiments and the image reconstruction program code structure 44).

Also illustrated in FIG. 1 is a generalized depiction of program/data store 50. In the program/data store 50, are stored program code structures (e.g., for non-contrast cardiac MRI dynamic myocardial perfusion analysis and visualization), as well as a related graphical user interface (GUI), operator inputs to same, etc., which are stored in computer readable storage media accessible to the various data processing components of the MRI system. As those in the art will appreciate, the program/data store 50 may be segmented and directly connected, at least in part, to different ones of processing computers of the system 20 having most immediate need for such stored program code structures in their normal operation (i.e., rather than being commonly stored and connected directly to the MRI system controller 22).

Indeed, as those skilled in the art will appreciate, FIG. 1 is a very high-level simplified diagram of a typical MRI system with some modifications so as to practice exemplary embodiments to be described hereinbelow. The system components can be divided into different logical collections of "boxes" and typically comprise numerous digital signal processors (DSP), microprocessors, special purpose processing circuits (e.g., for fast analog to digital (A/D) conversions, fast Fourier transforming, array processing, etc.). Each of those processors is typically a clocked "state machine" wherein the physical data processing circuits progress from one physical state to another upon the occurrence of each clock cycle (or predetermined number of clock cycles).

Not only does the physical state of processing circuits (e.g., CPUs (central processing unit), registers, buffers, arithmetic units, etc.) progressively change from one clock cycle to another during the course of operation, the physical state of associated data storage media (e.g., bit storage sites in magnetic storage media) is transformed from one state to another during operation of such a system. For example, at the conclusion of an MR imaging reconstruction process, an array of computer-readable accessible data value storage sites (e.g., multi-digit binary representations of pixel values) in physical storage media will be transformed from some prior state (e.g., all uniform "zero" values or all "one" values) to a new state. In the new state, the physical states at the physical sites of such an array (e.g., of pixel values) vary between minimum and maximum values to represent real world physical events and conditions (e.g., the tissues of a patient over an imaged region space). As those in the art will appreciate, such arrays of stored data values represent and also constitute a physical structure - as does a particular structure of computer control program codes that, when sequentially loaded into instruction registers and executed by one or more CPUs of the MRI system 20, cause a particular sequence of operational states to occur and be transitioned through within the MRI system.

The exemplary embodiments described below provide improved ways to acquire and/or process MRI data and/or to generate and display MR images.

First, the overview of the following MRI system according to the exemplary embodiment will be described. The MRI system according to the exemplary embodiment includes the MRI sequence controller 30 and the MRI system controller 22. The MRI sequence controller 30 performs, for multiple given times of different lengths, data acquisition for acquiring data on slices of a heart after the multiple given times since application of a tag pulse on a tag region. For example, the MRI sequence controller 30 acquires data on multiple apical to basal slices as the data on the slices of the heart. For example, the MRI sequence controller 30 acquires left ventricular short axis image data on multiple apical to basal slices for multiple different TIs (Time to Inversion).

The MRI system controller 22 (also referred to as "display controller") displays multiple results of analysis of the data acquired for the multiple given times such that a relationship between the multiple given times and the slices can be identified. In the exemplary embodiment, the MRI system controller 22 performs the analysis and controls display of the analysis results.

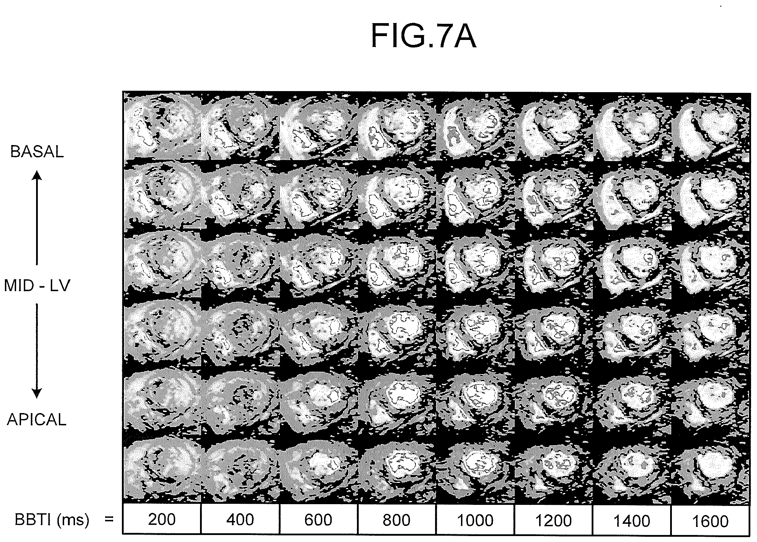

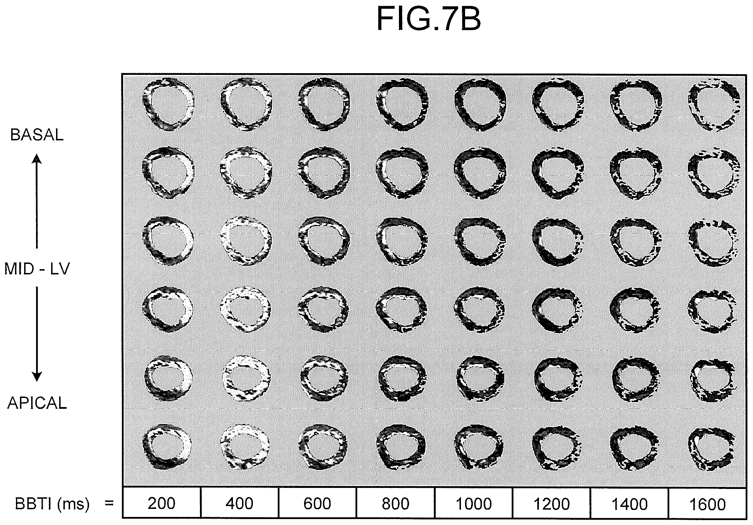

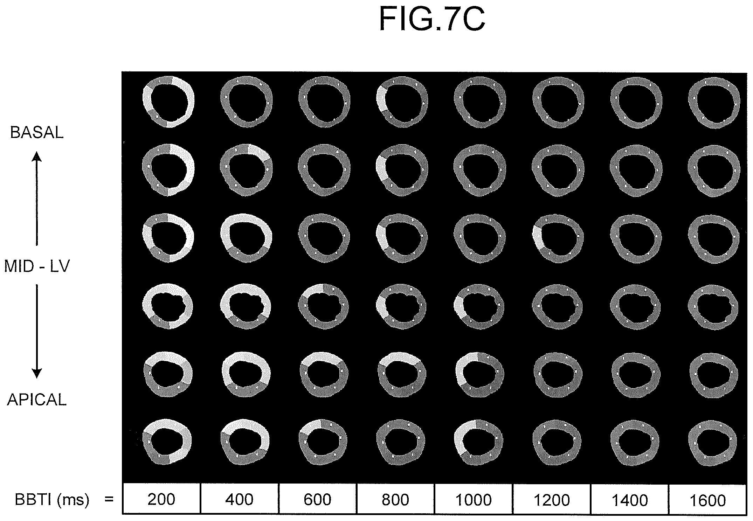

There are various methods for displaying such analysis results. For example, the MRI system controller 22 displays analysis images that are the results of the analysis of respective data sets in the one panel and in a matrix whose axes represent a direction of the multiple given times and a direction of the slices (see FIGS. 7A to 7C). The analysis images include images like those shown in FIG. 7A in which a color of different densities or different colors are allocated depending on signal intensity of each pixel on a pixel-by-pixel basis to the original image (tag-off, tag-on, or subtracted images), images like those shown in FIG. 7B in which a color of different densities or different colors are allocated depending on signal intensity of each pixel on a pixel-by-pixel basis to the images segmented so as to remove non-myocardial signals, images like those shown in FIG. 7C obtained by further segmenting the myocardium and in which a color of different densities or different colors are allocated depending on signal intensity of each segment on a segment-by-segment basis.

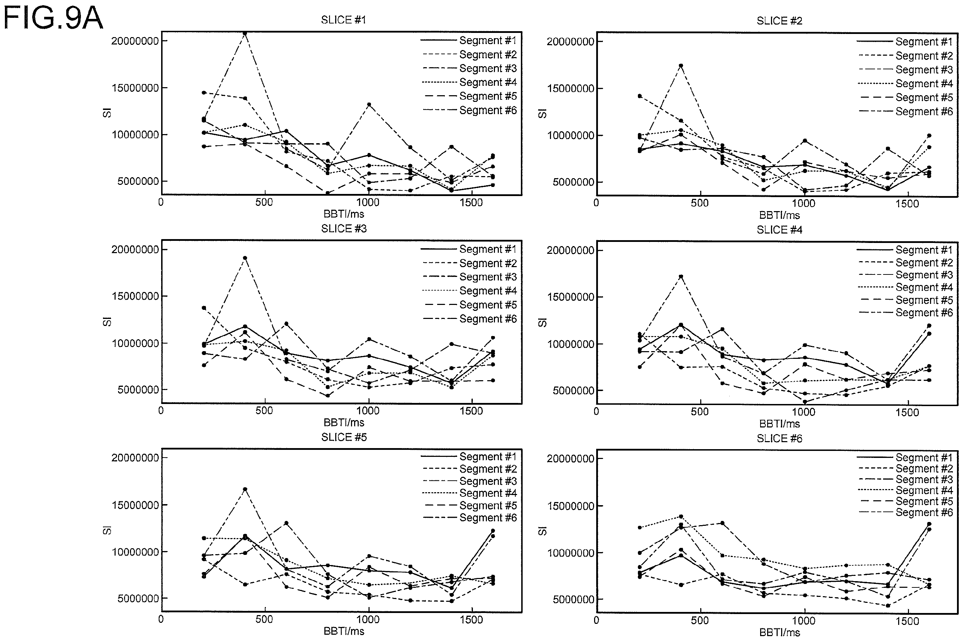

For example, the MRI system controller 22 displays, as the results of the analysis, a graph of perfusion curves representing changes of signal intensity in a direction of the given times, the graph being created by analyzing the data on the slices on a segment-by-segment basis (see FIGS. 9A to 9C). Here, the MRI system controller 22 can perform control for, upon accepting from an operator a selection operation regarding images (the original images or analysis images) displayed, displaying the graph of the perfusion curves corresponding to an image selected by the operator.

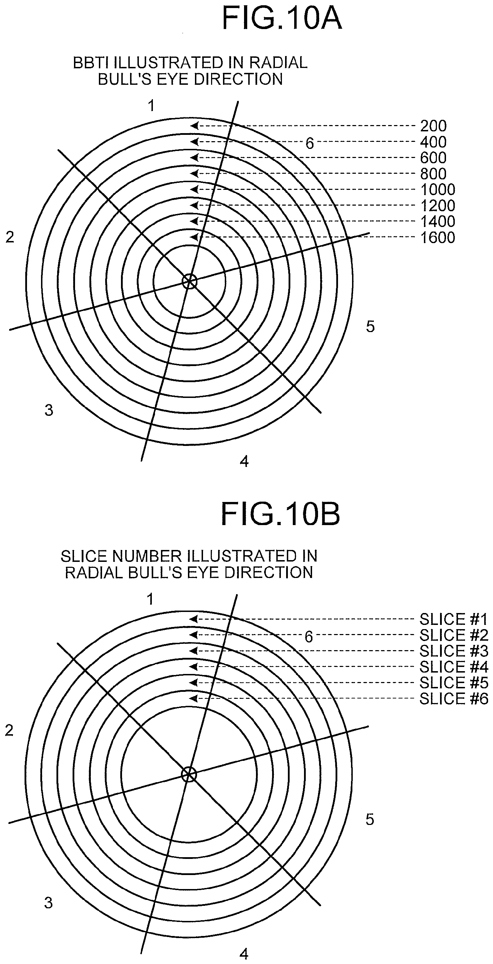

For example, the MRI system controller 22 displays, as the results of the analysis, a bull's eye map in which the data on the slices is analyzed on a segment-by-segment basis, in which different densities or different colors are allocated depending on signal intensity of each segment and which is created with a radial direction of concentric circles serving as a direction of the multiple given times.(see FIGS. 10A to 10B).

In the following exemplary embodiments, the above processes will be described as those performed by the MRI sequence controller 30 and the MRI system controller 22, but embodiments are not limited to this and other controllers may perform the processes.

Use of an gadolinium (Gd)-based contrast agent, possibly in association with a stress perfusion agent, is known, but use of such contrast agents is often not an acceptable MRI technique for detecting infarct and ischemic lesions in myocardium. LGE (Late gadolinium contrast enhancement) measures a difference between normal and infarct myocardium by observing T1-contrast (wash-out) of the gadolinium contrast agent via observed MRI signals from the myocardium after injection of the gadolinium contrast agent. In order to obtain a realistic observation of myocardium under stress, such stress may be induced by patient physical exercise or injected drug-induced stress (intended to cause increase in heart rate, dilation of cardiovascular blood vessels, etc., similar to that caused by physical exercise) so as to hopefully better observe blood flow abnormalities under such transient stress situations.

ASL (Arterial Spin Labeling) has also been used for non-contrast myocardial ischemia evaluation, but the object of this technique has been limited to a single slice at a single time point without the capability to generate perfusion curves or three-dimensional (3D) coverage. The corresponding analysis method is considerably different from the inventors' new non-contrast dynamic MRI perfusion analysis and visualization.

In particular, the inventors have now discovered a way to avoid use of such contrast agents. The way is to achieve a non-contrast (i.e., without injection of a chemical contrast agent) MRI technique for producing myocardial perfusion curve/table data that can be used to distinguish between normal myocardium, ischemic myocardium and infarct myocardium. Indeed, even revascularized infarct myocardium (treated with revascularization techniques such as operations for stent insertions into blood vessels, surgical bypass blood vessel operations, etc.) may be distinguished. In accordance with exemplary embodiments, such myocardium distinctions can be made within any desired region of interest (e.g., an operator-defined arbitrary region of interest, a standard AHA segment, a single pixel, etc.).

Although injection of a contrast agent (e.g., gadolinium-based) can be avoided, it may still be desirable to use exemplary embodiments in combination with patient stress (either exercise-induced or drug-induced) so as to better detect abnormalities that may exist or be more prominent only during such patient's stressed situations.

In exemplary embodiments, a set of "tag-on" (2D or 3D) MRI k-space data is acquired after being "tagged" with an initial spatially selective RF pulse (e.g., typically a spatially selective 180° inversion pulse) where the data acquisition subsequence starts after a given time to inversion time delay (TI). A similar set of "tag-off" MRI data is also acquired using the same TI delay interval - but without the initial spatially selective RF tagging pulse. This technique is sometimes known in the art as BBTI (Black Blood Time to Inversion) imaging. A sequence of such tag-on/tag-off data sets for each of multiple TI times is acquired in k-space.

For each given TI time, 2D/3D Fourier Transform reconstructed spatial domain tag-on and tag-off image data sets (i.e., the result of well known 2DFT/3DFT reconstruction processes) are subtracted (e.g., on a pixel-by-pixel basis) to provide BBTI blood perfusion images where blood perfusion (MR signal strength) as a function of time can be plotted or tabulated. For any given region of interest (e.g., an AHA segment, arbitrary operator-defined ROI, or a single pixel), a graph where data values are plotted with respect to the time axis provides a blood perfusion curve as a function of time. The function of time provides several dimensions of differentiation between perfusion curves for "normal" myocardium, abnormal ischemic myocardium, infarct myocardium, and revascularized (treated) ischemic myocardium. As will be expected, infarct myocardium shows no perfusion (i.e., no peak or increase in detected MRI signal strength). However, ischemic myocardium has a time-delayed peak flow time of occurrence, as well as less detected signal intensity, when compared to normal (or revascularized) myocardium. Accordingly, the locations of ischemic lesions can be identified based upon time and/or amplitude (or even integrated area under the curve) comparisons and/or comparisons to predetermined thresholds, etc.

A perfusion curve and/or a corresponding data table pertaining to a region of interest (e.g., a predetermined AHA myocardium segment or an arbitrary operator-indicated ROI or a single pixel) that includes distinguishing characteristics (e.g., as displayed to an operator or stored data for later display to others) can differently depict the ROI as representing normal, ischemic, infarct or even revascularized myocardium - all without use of any injected contrast agent (e.g., gadolinium contrast agent).

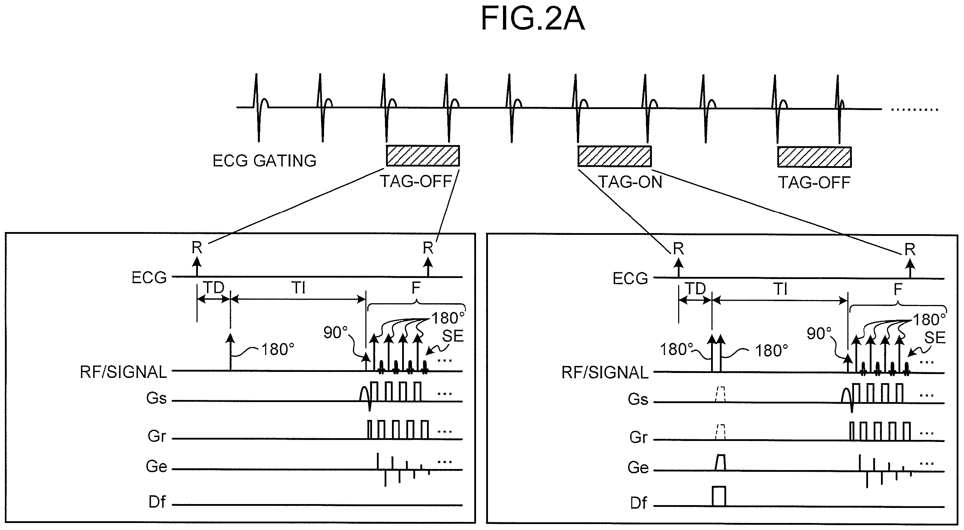

FIG. 2A is a schematic depiction of a first exemplary tag-on, tag-off MRI data acquisition sequence for use in the MRI system according to the embodiment. In the representative data acquisition sequence depicted at FIG. 2A, ECG synchronization is used so as to synchronize both "tag-off" and "tag-on" subsequences as depicted in FIG. 2A. In the tag-off subsequence shown to the left-lower side of FIG. 2A, a short initial fixed time delay TD is employed to ensure that the data acquisition subsequence begins during a desired diastole portion of the RST ECG signal. After the time delay TD, a non-selective 180° RF pulse is employed (to help suppressing background signals from a subsequent different image because the signal from myocardium is relatively small, e.g., about 10% of the total MRI signal). The actual data acquisition subsequence may, if desired, begin with a preparation pulse (e.g., a fat suppression pulse), followed by a desired data acquisition subsequence F initiated with a slice-selective (e.g., α° - typically 45° or 90°) NMR pulse to initiate a desired MRI data acquisition subsequence F, e.g., a sequence of slice-selective 180° RF refocusing pulses to elicit intervening RF spin echo (SE) responses during readout gradient Gr pulses. Each spin echo is preceded by a phase encoding Ge magnetic gradient pulse (which is varied for different echoes so as to elicit data for a respectively corresponding line in k-space). As those in the art will appreciate, such known MRI data acquisition subsequences might be, e.g., of the bSSFP (balanced Steady-State Free Precession) type (presently preferred) or of the fast spin echo (FSE) type or possibly of other types.

The tag-on data acquisition subsequence depicted at the right-lower side of FIG. 2A is similar to the tag-off subsequence - except that, after the time delay TD, a selective "tagging" 180° RF pulse (e.g., perhaps pulse at an oblique angle as represented by the dotted concurrent gradient pulses and the Df frequency offset pulse as depicted in FIG. 2A) is also used. As those in the art will appreciate, this will, in effect, revert a predetermined inflowing volume of blood back to a noninverted magnetization orientation - thus "tagging" this inflowing volume of blood. Accordingly, it will generate different MR signal responses than those for the tag-off subsequence as the RF tagged flowing blood MR nuclei enter the downstream ROI.

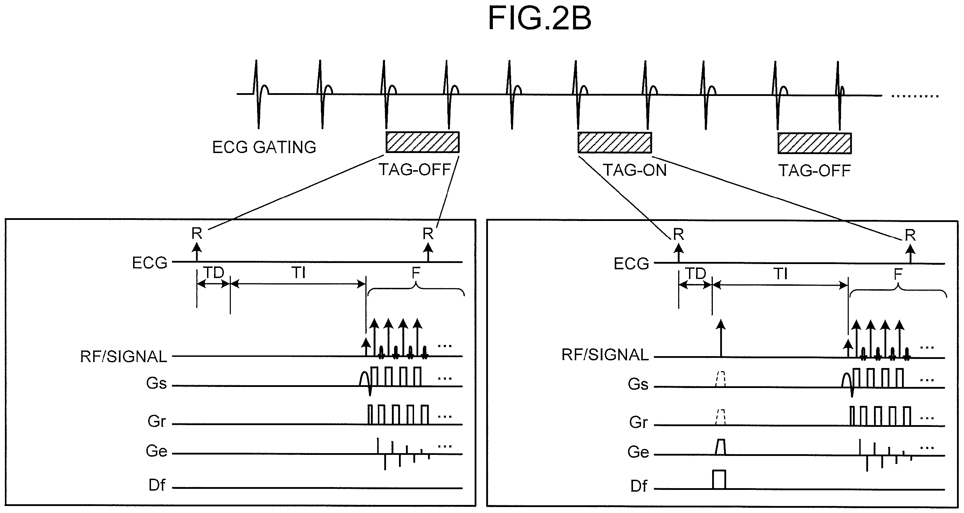

FIG. 2B is a schematic depiction of a second exemplary tag-on, tag-off MRI data acquisition sequence for use in the MRI system according to the embodiment. The tag-on/tag-off alternate acquisition subsequences depicted in FIG. 2B are the same as those depicted in FIG. 2A, except that, as will be observed, there is no initial non-selective 180° pulse (in either tag-on or tag-off subsequences) for background suppression purposes.

As depicted in dotted lines in both FIGS. 2A and 2B, to achieve a desired oblique angle for the spatially selective 180° tag-on pulse, there may be concurrent usage of Gs, Gr and Ge magnetic gradient pulses of differently chosen intensities.

MRI data acquisition of the exemplary embodiment has been described above and this will be described again. In the exemplary embodiment, the MRI system acquires a set of images (a set of myocardial perfusion images) where the movement of blood supplied to myocardial tissues is depicted by using a blood labeling (tagging) method, i.e., a non-contrast technique. The MRI system then analyzes the acquired set of images and displays the results of analysis to visualize them.

The method of labeling blood will be described simply. For example, when acquiring images depicting blood supplied to myocardial tissue, the MRI system applies an inversion pulse for inverting the longitudinal magnetization by 180° to an upstream blood-flowing region of the myocardial tissues as a tag pulse for labeling the blood. The blood to which the inversion pulse has been applied has a signal intensity allowing the blood to be distinguished from other bloods and background tissues in the course of longitudinal magnetization recovery. The length of time between the inversion pulse application timing and MR signal acquisition timing (i.e., acquisition timing at the k-space center line) is typically proportional to the distance traveled by the blood to which the inversion pulse is applied. Thus, by acquiring MR signals while changing the length of time between the inversion pulse application timing and the MR signal acquisition timing, a set of images depicting the movement of blood flowing into/out of the myocardial tissues can be acquired. There is another method where a non-selective pulse without selecting a region is applied, almost simultaneously with the inversion pulse, to invert the longitudinal magnetization of the whole imaging region by 180°. In this case, the upstream blood in the myocardial tissues is subject to twice applications of inversion pulses and accordingly has a signal intensity that allows the blood to be discriminated from other bloods and background tissues. The method where a non-selective pulse is applied is shown in FIG. 2A and the method where no non-selective pulse is applied is shown in FIG. 2B. Tag pulse is limited to inversion pulse for inverting the longitudinal magnetization by 180°.

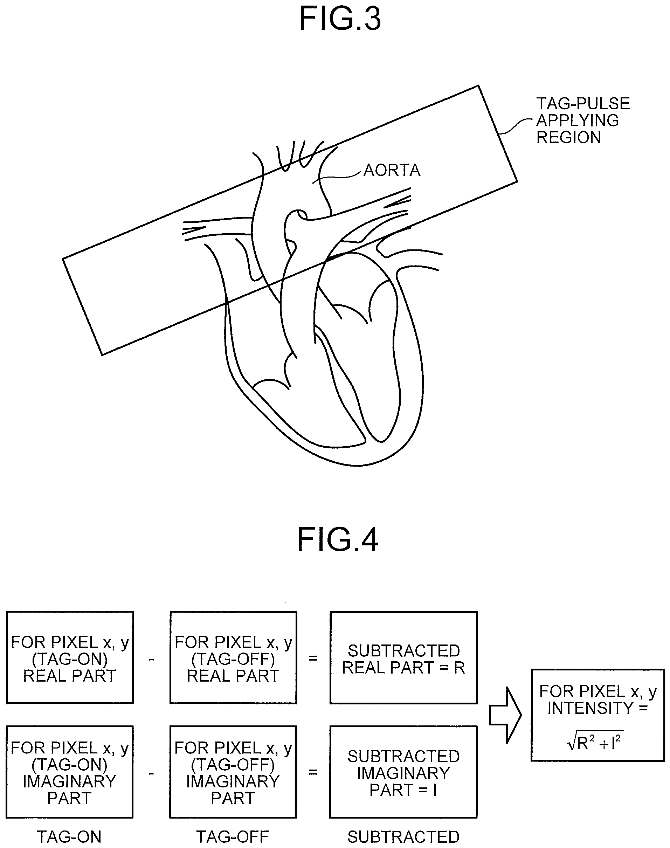

FIG. 3 depicts a region to which tag-pulses are applied according to the embodiment. Blood is sent from the left ventricle of the heart to the whole body via the aorta shown in FIG. 3. Although it is not shown in FIG. 3, the arteries branching from the aorta to the right and left are the right and left coronary arteries, and the left coronary artery further bifurcates into a blood vessel descending forward and a blood vessel coming around to the back. Blood is supplied to myocardial tissues via three blood vessels: the right coronary artery (RCA), the left anterior descending coronary artery (LAD), and the left circumflex coronary artery (LCX). Because the embodiments are aimed at capturing and analyzing movement of blood flowing into and then flowing out of myocardial tissues, the region to which a tag pulse is applied is preferably set around the aortas in the upper part of the heart as shown in FIG. 3.

In the exemplary embodiment, not only tag-on image data where blood is labeled but also tag-off image data where blood is unlabeled are acquired and subtracted images therebetween will be analyzed, because subtraction between tag-on and tag-off image data makes it possible to analyze more accurately the blood where the tag pulses are applied, a consequence of signals other than of the blood where tag pulses are applied having been subtracted.

Because both tag-on image data and tag-off image data are all acquired over a series of pulse sequences without standby time (without operational interposition by the operator) in the method of the embodiment, its subsequences are referred to as "tag-on data acquisition subsequence", "tag-off data acquisition subsequence", etc. The difference between FIGS. 2A and 2B is in whether to once invert (FIG. 2A) or not to invert (FIG. 2B) the longitudinal magnetization over the whole imaging region. In the former case, background signals are low. In view of subsequent generation of subtracted images between tag-on and tag-off images, the former method with smaller effects of misregistration between tag-on and tag-off images is preferable.

As will be observed in comparison between the left and right views in FIG. 2A or the left and right views in FIG. 2B, the difference between "tag-on data acquisition subsequence" and "tag-off data acquisition subsequence" is in whether to apply selective inversion pulses. As shown in FIG. 2A, in a case where non-selective pulses are also applied, a non-selective pulse and a selective pulse are typically applied almost simultaneously. Thus, "TI" representing, in FIGS. 2A and 2B, the time since the non-selective pulse application timing until the data acquisition start timing may be considered as the approximate time since the selective pulse application timing until the data acquisition start timing.

In the embodiment, the MRI sequence controller 30 preferably acquires all sets of tag-on and tag-off image data for multiple slices during one breath-holding (e.g. 20 seconds). For example, the MRI sequence controller 30 acquires slice images of the left ventricle (LV) from the ventricular apex to the ventricular base for three slices. Thereafter, the MRI system acquires tag-on and tag-off image data sets for six slices by interpolation processing.

In the embodiment, the MRI sequence controller 30 executes above-mentioned alternate tag-on/tag-off subsequences for different lengths of multiple TIs. For example, the MRI sequence controller 30 executes alternate tag-on/tag off subsequences for the respective TIs: TI=200 ms, TI=400 ms, TI=600 ms, TI=800 ms, TI=1,000 ms, TI=1,200 ms, TI=1400 ms, and TI=1600 ms. The subsequences for different TIs may be executed as a series of pulse sequences without standby time (without operational interposition by the operator) or may be executed with appropriate insertion of standby time. The order of TIs can be arbitrarily changed.

It can be also properly changed as to which TI or which interval between the TIs is used for the acquisition. These TIs may be set by accepting inputs from an operator on a GUI for accepting imaging conditions, or set in one of pre-sequences. Normally, examinations using an MRI system include a set of imaging sequences for acquiring images for various types of diagnosis and a set of pre-sequences executed prior to the set of imaging sequences, and a series of these sets of sequences are sequentially executed successively with operator's operations being interposed, etc. The pre-sequence for setting TIs is for setting appropriate TIs to be used for image sequences later in a way that, for example, MR signals respectively for different TIs are acquired respectively for different TIs while changing the TI, the MR images are concurrently displayed on the GUI to allow the operator to select an image, and a profile of a line ROI (region of interest) on an MR image is obtained. Such a pre-sequence can be referred to as BBTI-prep etc. The sets of pre-sequences include, in addition, a sequence for acquiring a registration image, a sequence for adjusting non-uniformity in the magnetic field, a sequence for acquiring a coil sensitivity map, etc.

In the embodiment, the MRI sequence controller 30 has the same cardiac phase for data to be acquired between subsequences for different TIs. For example, the MRI sequence controller 30 acquires data for any TI in a diastolic cardiac phase. In this case, non-selective pulse and selective pulse application timing can be obtained by calculating back by TIs from the data acquisition timing.

In the embodiment, the method of acquiring separately a set of tag-on image data and a set of tag-off image data has been described. However, embodiments are not limited to this and any method is satisfactory as long as non-contrast myocardial tissue perfusion images are acquired. In other words, acquiring a set of tag-off image data is not a necessary configuration. In the embodiment, the method of alternately acquiring a set of tag-on image data and a set of tag-off image data for each slice or each slice encode has been described, but embodiments are not limited to this. For example, a set of tag-off image data may be acquired after a whole set of tag-on image data is acquired, or vice versa. In the embodiment, the method for acquiring a set of tag-off image data for all TIs has been described but embodiments are not limited to this. A set of tag-off image data acquired for one TI may be used to calculate subtracted images for another TI.

In the embodiment, the method of successively acquiring sets of image data for different TIs in a series of pulse sequences without standby time (without operational interposition by the operator) has been described, but embodiments are not limited to this. For example, a standby time may be inserted every time when the TI is changed. In the embodiment, the method of acquiring image data in the diastolic cardiac phase in synchronization with an ECG synchronization signal has been described, but embodiments are not limited to this and image data may be acquired in another cardiac phase. Furthermore, the acquiring methods of the embodiment shown in FIGS. 2A, FIG. 2B, and FIG. 3 are not limited to them and may be changed arbitrarily.

The methods used by the MRI system to acquire a set of non-contrast myocardial perfusion image data have been described above. Hereinafter, an example will be described where the MRI system or an image processing apparatus different from the MRI system analyzes a set of myocardial perfusion image data acquired by the MRI sequence controller 30 etc. The inventers now propose exemplary analysis methods that can be perhaps best suited for use with our non-contrast dynamic myocardial perfusion techniques. For example, the new methods can process a dynamic, 3D image data set with the capability to visualize blood perfusion in the left ventricle (LV) and to show perfusion curves for any segments or ROI defined by the operator.

As will be understood by those in the art, our perfusion analysis and visualization methods can be incorporated into the MRI system of FIG. 1 or, alternatively, may be practiced as part of a separate image analysis/display system remotely located from the MRI system of FIG. 1 where original tag-on and tag-off image data are acquired.

For dynamic 3D images obtained from our non-contrast perfusion techniques, we propose the following exemplary presently preferred analysis procedures (not all of which may always be required or desired):
1. Perform complex data subtraction between tag-on and tag-off images.
2. Perform image registration: rigid or non-rigid registration for 3D images at different BBTI.
3. Create histograms of tag-on and tag-off images to check for myocardium signal loss caused by susceptibility and/or by tagging slice affecting the imaged slice. By subtraction of histograms, adverse susceptibility and tagging slice effects on the imaged myocardium can be detected.
4. Use of myocardial segmentation.
5. Display of the segmented myocardium in a color map format.
6. Create a perfusion curve for each segment or ROI across all slices.
7. Concurrent display of all 3D slices versus BBTI.
8. Concurrent automatic display of respectively corresponding perfusion curves when selecting a segment and/or ROI.
9. Perfusion curve fitting for quantification purposes.

### (1: Creation of subtracted image)

The MRI system controller 22 performs complex-valued subtraction operation on a pixel-by-pixel basis on tag-off and tag-on image sets that are acquired for the same slice and at the same BBTI. As described above, in the embodiment, tag-on and tag-off images are acquired alternately during a breath-hold and the cardiac phase is also acquired and thus it is considered that registration is not necessary in performing subtraction, but embodiments are not limited to this. Registration between tag-on and tag-off images may be performed before subtraction operation. FIG. 4 illustrates subtraction between tag-on and tag-off images on a pixel-by-pixel basis according to the embodiment. As shown in FIG. 4, the MRI system controller 22 performs an arithmetic for subtracting the real part of a tag-off image from the real part of a tag-on image on a pixel-by-pixel basis. the MRI system controller 22 also performs an arithmetic for subtracting the imaginary part of the tag-off image from the imaginary part of the tag-on image on a pixel-by-pixel basis. The subtracted image should be in complex format (real and imaginary parts, R + jI) because the intensity of subtracted pixels is then made insensitive to signal changes caused by possible phase shifts between tag-on and tag-off signals. Thus, the original tag-on and tag-off images should also be in complex-valued format. The complex-valued data after subtraction is then used to build an intensity image for perfusion analysis.

The reason why complex-valued subtraction operation is performed in the embodiment will be described here. The magnetization vector of the atomic nuclei is represented by the real number component (in-phase component) and the imaginary number component (quadrature phase component) on the complex plane. Thus, in MRI, k-space data for each of the real and imaginary number components is acquired, real and imaginary number images are generated by Fourier Transform, and then amplitude images and phase images that are absolute-value images are generated.

For example, tag-on and tag-off images have a difference in whether a selective pulse is applied. This difference appears as a difference in the longitudinal magnetization component, but it appears as a difference of phase of transverse magnetization component on the x-y plane after application of excitation pulse. This is because, for example, the phase differs between a case where the upward magnetization vector inclines toward the x-y plane and the case where the downward magnetization vector leans to the x-y plane. Thus, in the embodiment, in order to take the phase difference into consideration correctly, the MIR system controller 22 performs complex-valued subtraction operation.

### (2: Regarding image registration)

In the embodiment, multiple slice images of the left ventricle are acquired for the same BBIT (the slice images of the left ventricle may be partly created by interpolation processing) and slice images of the left ventricle at different positions are acquired at different BBTIs. Since the 3D images at different BBTIs are acquired at different acquisition times, registration between different BBTI images may be necessary. The MRI system controller 22 thus performs registration between slice images of the left ventricle at the same BBTI and also between slice images of the left ventricle at different BBTIs. Note that embodiments are not limited to this and both or one of such registrations may be omitted.

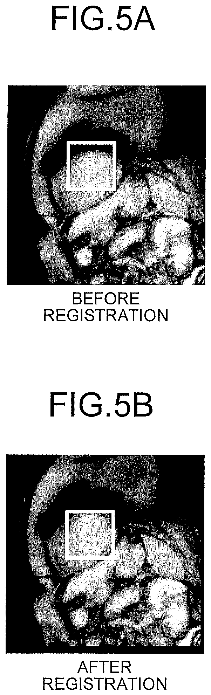

FIG. 5A illustrates left ventricle myocardium intensity images at different BBTIs before registration according to the embodiment. FIG. 5B illustrates left ventricle intensity images at different BBTIs after registration according to the embodiment. The images in FIGS. 5A and 5B demonstrate the 3D registration for an imaging slab across all BBTIs (for simplicity in explanation, only one slice is shown).

The MRI system controller 22 performs rigid or non-rigid registration. In other words, registration is not limited to rigid registration. For example, the donut shape of a left ventricle slice image at one BBTI may have a slightly different shape at another BBTI. In this case, non-rigid registration should be performed.

In the proposed method, left ventricle registration is preferable. FIGS. 5A and 5B illustrate examples where a registration window is placed on the left ventricle only. When a registration window is placed on the left ventricle only, local registration for the left ventricle is allowed. For example, the MRI system controller 22 displays at least one left ventricle slice image among a set of left ventricle slice images at different BBTIs and also displays a registration window (e.g. a box) and accepts an operation from the operator. For example, the operator moves the registration window to a position on the left ventricle slice image such that the window includes the left ventricular myocardium. Subsequently, the MRI system controller 22 displays a left ventricle image at another BBTI and also displays a registration window in the position that has been set by the operator. If the registration window displayed here does not include the left ventricle, the operator, for example, then moves the left ventricular image such that the left ventricular myocardium image is within the registration window. For example, by repeating such processing, the MRI system controller 22 performs registration between left ventricle slice images at different BBTIs. It can be seen that the myocardium in a slightly lower position with respect to the registration window in FIG. 5A before registration is almost within the center of the registration window in FIG. 5B after registration. The registration processing is an example only and registration may be performed, for example, by automatic processing using image. Such method may be applied to left ventricle slice images at the same BBTI.

If a non-selective pulse is used, the contrast between a heart chamber and the surrounding myocardium will be inverted at some BBTIs. In this case, both tag-on and tag-off images should be utilized in the registration process. For example, one can select the images with positive contrast (myocardial signal intensity SI > LV chamber blood SI) and perform registration on them. Then one can select negative contrast images and perform another registration process. There is a significant blood exchange in the heart chamber. Thus, as can be seen with reference to FIG. 7A, the signal value in the heart chamber may be high or low depending on the BBTI, i.e., depending on the BBTI, the myocardial signal intensity SI may be higher than the heart chamber blood signal intensity SI (positive contrast) or the heart chamber blood signal intensity SI is higher than the myocardial signal intensity SI (negative contrast). It is preferable that, if a reference image for registration is selected, an image with an optimum contrast (positive contrast or negative contrast) be selected according to the BBTI. The registration shift of pixels of each image relative to the reference images can be recorded for a combined registration process. Manual shifting with a visual check may be necessary to achieve the best registration.

### (3: Check for myocardium signal loss)

Check for myocardium signal loss will be described here. The myocardium signal loss checking process is not necessarily performed at this stage, and it may be performed in parallel with a different process or at an arbitrary time, e.g. before or after a different process (e.g. before 1: a subtracted image is generated, or 2: image registration). In other words, when the MRI system controller 22 detects that there is a myocardium signal loss, the MRI system controller 22 outputs the fact as information in association with the images and analysis results to be later provided (analyzed images, graphs, bull's eye maps etc.) to the operator, thus drawing attention of a person (e.g. a doctor) who browses the images or analysis results (e.g. not to confuse myocardium signal loss with lesion). There are some types of confuse myocardium signal loss: in one type, an artifact occurs when an imaging region (imaging slab) and a tag region to which a tag pulse (selective pulse in the embodiment) is applied overlap; and in another type, an artifact occurs as a result of a difference in magnetic susceptibility.

For example, the MRI system controller 22 sets a line ROI traversing the left ventricle on a tag-off, tag-on, or subtracted image and acquires a signal profile on the line ROI (this signal profile is referred to as "histogram" above). On the basis of the signal profile, the MRI system controller 22 determines whether there is a myocardium signal loss (e.g. a local decrease of signal value). The MRI system controller 22 sets a line ROI over the whole image (discretely, as appropriate) and determines whether there is a myocardium signal loss. The MRI system controller 22 may determine whether there is a myocardium signal loss by using a signal profile obtained by performing subtraction between a signal profile on a tag-on image and a signal profile on a tag-off image.

When the MRI system controller 22 determines that there is a myocardium signal loss, the MRI system controller 22 adds this information to an image on which the determination has been made, thereafter displays the loss information as reference information when displaying images and analysis results, etc. Determination on whether there is such a loss is not limited to automatic methods, and manual methods where, for example, signal profiles are displayed to allow an operator's visual check may be used. Furthermore, an example has been described where a line ROI is set on a tag-off, tag-on, or subtracted image, but embodiments are not limited to this. For example, a line ROI may be set on a segmented image like that described below. Alternatively, if, for example, cine images are previously acquired as another series among series of pulse sequences, for example, the MRI system controller 22 displays the cine images as video images to allow the operator to check visually whether there is a loss. Here, the method where loss information is displayed as reference information when it is determined that there is a myocardium signal loss, but this does not put any limitation. For example, the MRI system controller 22 may correct the target image when generating the above-described subtracted image or performing image registration by using this loss information.

### (4: Myocardial segmentation)

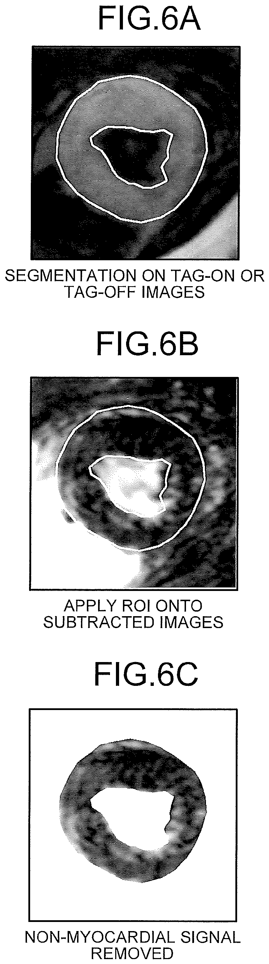

FIG. 6A illustrates segmentation imposed on tag-on or tag-off images according to the embodiment. FIG. 6B illustrates a region of interest to be composited into a subtracted image according to the embodiment. FIG. 6C illustrates a region of interest to be composited into a clear image according to the embodiment where non-myocardial signals outside the segmented volume have been removed.

LV segmentation can be achieved after registration by aligning the LV along successive BBTIs as depicted in FIGS. 6A, 6B and 6C. On the tag-off images, the endocardial and epicardial contours may be drawn (manually or semi-automatically) for each slice and saved. Then the saved contours can be applied onto the subtracted images. To visualize LV myocardium only, other signals can be removed (e.g., see the "clear" image of FIG. 6C). Note that the LV contours should be carefully placed to exclude any effects resulting from artifacts (e.g., susceptibility artifacts) and tagging slice interference.

In other words, as illustrated in FIG. 6A, the MRI system controller 22 accepts endocardial and epicardial contours manually specified by the operator on a tag-on or tag-off image and thus performs segmentation. Alternatively, the MRI system controller 22 performs image processing on the tag-on or tag-off image to extract the endocardial and epicardial contours from the tag-on or tag-off image.

As illustrated in FIG. 6B, the MRI system controller 22 puts the endocardial and epicardial contours, which are acquired from the tag-on or tag off image, on the subtracted image. As illustrated in FIG. 6C, the MRI system controller 22 then uses the endocardial and epicardial contours to remove non-myocardial signals from the subtracted image, i.e., the MRI system controller 22 removes the inner region of the endocardium and the outer region of the epicardium from the subtracted image. At this stage, for example, when the operator finds a myocardium signal loss, for example, the MRI system controller 22 accepts an contour adjusting operation (e.g. for adjusting the contours to remove the loss parts) from the operator so that the myocardium signal loss can be removed from the analysis target. For manually specifying (or automatically extracting) endocardial and epicardial contours, the MRI system controller 22 can appropriately select an image in which the contours are easily specified (or easily extracted) (e.g., an image with significant positive or negative contrast) among tag-on or tag-off images.

### (5: Myocardial color map display)

FIG. 7A depicts an exemplary perfusion visualization according to the embodiment wherein LV slice images before segmentation are displayed together in one display panel as a function of BBTI time periods. FIGS. 7B and 7C depict exemplary perfusion visualizations according to the embodiment wherein LV slice images after segmentation are displayed together in one display panel as a function of BBTI time periods. Perfusion visualization can be achieved as shown in FIGS. 7A to 7C. Here, LV images of apical to basal slices at all BBTIs are displayed in one panel. In this one panel display, the blood perfusion in the left ventricle can be observed (a color map is preferably used to better visualize the signal intensity change). FIG. 7A depicts visualization before LV segmentation. FIGS. 7B and 7C depict visualization after segmentation.

In other words, as depicted in FIGS. 7A and 7B, the MRI system controller 22 allocates colors corresponding to signal values on a pixel-by-pixel basis for each of LV slice images before or after segmentation to generate color map images. The MRI system controller 22 then displays all color map images in one panel and in a matrix whose horizontal axis represents the BBTI direction and the vertical direction represents the apical to basal slice direction. One panel display methods are not limited to this. For example, the horizontal and vertical axes may be transposed to each other. For example, according to specifications by an operator, rows and columns may be displayed as partly omitted.

Furthermore, in the above example, the method of allocating colors corresponding to signal values on a pixel-by-pixel basis has been described, but embodiments are not limited to this. As illustrated in FIG. 7C, the MRI system controller 22 can further segment the myocardium and allocate colors corresponding to the signal values in each of the segments (e.g. the mean value), thereby generating a color map. When displaying color map images of FIGS. 7A to 7C, the MRI system controller 22 can together display a color bar indicating the relationship between color allocation and signal intensity.

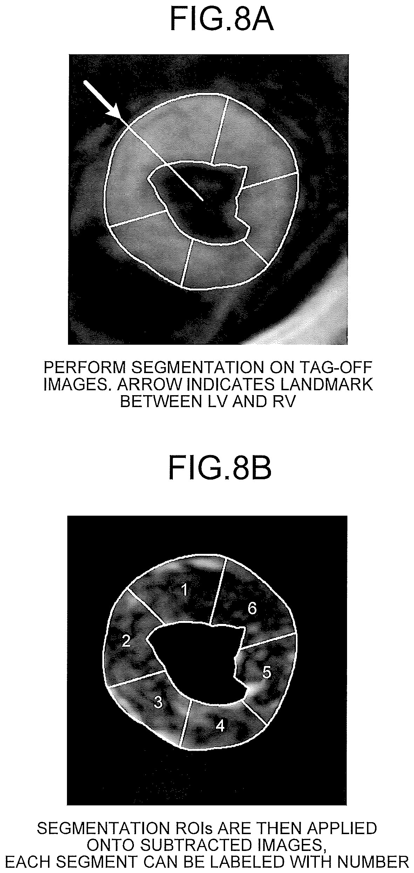

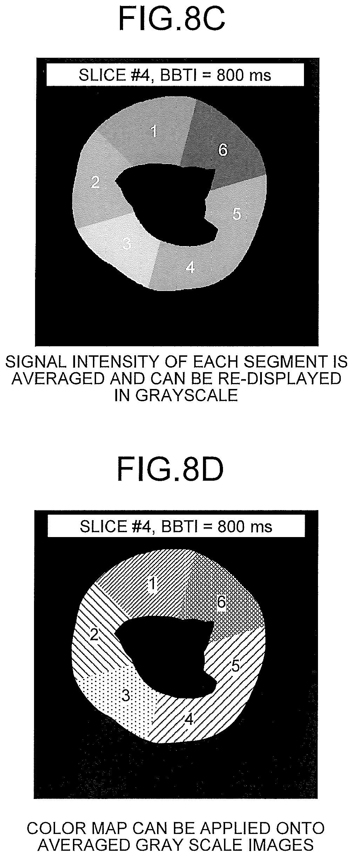

FIGS. 8A to 8D depict coronary artery territory segmentation in the left ventricle according to the embodiment, where an AHA six-segment model is used to acquire a color-mapped display of the segmented exemplary coronary artery region of the left ventricle. For example, coronary artery territory segmentation in LV may, for example, be either the standard AHA six-segment model or, if desired, any number of other operator-defined segmentation. The AHA six segmentation depicted in FIGS. 8A, 8B, 8C and 8D starts from the groove between LV and RV (right ventricle) and automatically runs clockwise. That is, the groove is marked as the start point to identify the coronary artery territory. Each succeeding numbered AHA segment can be labeled, averaged and color-mapped to distinguish and show intensity changes among all segments as depicted in FIGS. 8A to 8D.

As described above, blood is supplied to myocardial tissues via three blood vessels: the right coronary artery (RCA), the left anterior descending coronary artery (LAD), and the left circumflex coronary artery (LCX). Among myocardial tissues, Segments 1 and 2 are supplied with blood via the left anterior descending coronary artery, Segments 2 and 3 are supplied with blood supply via the right coronary artery, and Segments 5 and 6 are supplied with blood supply via the left circumflex coronary artery.

For example, as depicted in FIG. 8A, the MRI system controller 22 segments the whole myocardium on a tag-on or tag-off image by operator's manual operation, or automatically, into AHA six segments. In automatic segmentation, for example, segmentation is performed using a landmark present between LV and RV as a start point. Subsequently, as depicted in FIG. 8B, the MRI system controller 22 applies a six-segment boundary acquired from a tag-on or tag-off image to a subtracted image and eliminates non-myocardial signals from the subtracted image.

The MRI system controller 22 then analyzes the signal values on a segment-by-segment basis. In other words, for example, the MRI system controller 22 first analyzes signal values on a pixel-by-pixel basis and calculates a mean value of analysis results of the respective pixels on a segment-by-segment basis, thereby calculating analysis results on a segment-by-segment basis. The calculation of a mean value is a mere example. As depicted in FIGS. 8C and 8D, the MRI system controller 22 allocates colors corresponding to the signal values (e.g. mean value) in the segments on a segment-by-segment basis to generate a color map image. FIG. 8C depicts in fact an example of a display in grayscale (in gradations of gray), to say it a "color" map and FIG. 8D is an example of a color map image to which different colors are allocated. The MRI system controller 22 may display color map images generated as described above in FIGS. 7C in one panel. As depicted in FIGS. 8C and 8D, the MRI system controller 22 can display, for each of the color map images, the slice numbers, BBTI value, identification number of each segment, etc, as appropriate.

### (6: Creation of perfusion curves for each ROI)

The MRI system controller 22 creates and displays a graph representing changes in the signal value over time on a segment-by-segment basis. FIGS. 9A to 9C depict simultaneous one-panel display visualization of perfusion curves for each segment in each of different slices as a function of BBTI values using different exemplary visualization presentations according to the embodiment. Perfusion curves of each segment in each different slice along the different BBTI images can be created as shown in FIGS. 9A to 9C. With the saved ROIs of all segments, the perfusion curves can be automatically generated for all segments in all slices. The FIG. 9A example shows unsmoothed raw perfusion curves from one patient. The perfusion curves can be also generated from any ROI that the operator has specified.

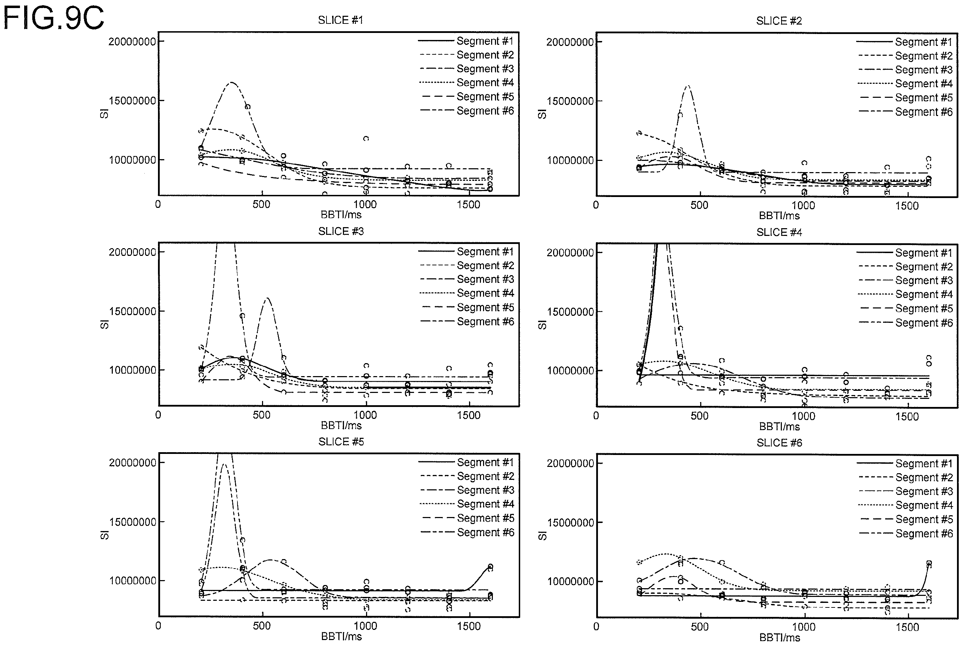

Conventional polynomial curve fitting or curve smoothing techniques may be applied to assist in further quantification analysis as shown in FIG. 9C. Preferably, two curve fitting equations are used to best fit the perfusion curves. The fitted parameters should be able to describe the perfusion peak intensity, timing of the peak, and the area under the peak. Preferably any identifiable abnormal parameters (e.g., as ascertained by quantitative analysis of the curve fitted perfusion data) should be marked in a fitted parameter table and/or directly on the displayed perfusion curves. Similarly, any region(s) corresponding to the detected abnormalities preferably should be marked directly onto the visualizations of the corresponding AHA segments.

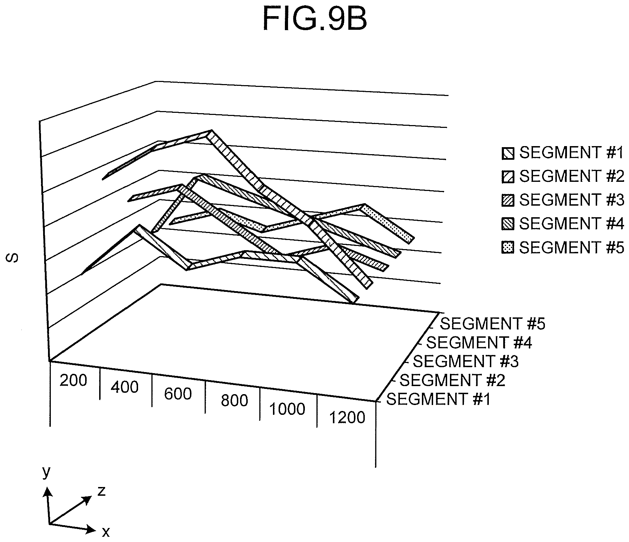

FIG. 9B illustrates a simulated 3D visualization of perfusion curves where (using a typical orientation of orthogonal x,y,z coordinate axes) BBTI values are plotted with respect to an x-axis, relative signal intensity (perfusion) is plotted with respect to a y-axis and slice number is plotted with respect to a z-axis of the visualization display.

Although the perfusion curves are shown in one panel in FIGS. 9A to 9C, any one perfusion curve can be fetched for overlaid display in FIG. 7A or FIG. 7B when a particular respectively corresponding slice or segment is selected by an operator (e.g., by "clicking" a mouse arrow when positioned over that selected slice or segment of a particular slice at a particular BBTI).

In other words, the MRI system controller 22 analyzes the signal values of each LV slice image on a segment-by-segment basis and creates curves representing changes in the signal value along the BBTI direction on a segment-by-segment basis. For example, as shown in FIGS. 9A and 9C, the MRI system controller 22 displays arrayed perfusion curves of six slices in one panel. The MRI system controller 22 can express differences of perfusion curves between each of the segments by differences of colors or the types of lines. Furthermore, for example, if the MRI system controller 22 accepts a selection of given LV slice image on the displays of FIGS. 7A to 7C when the MRI system controller displays FIGS. 7A to 7C, the MRI system controller 22 may display a graph corresponding to the accepted LV slice image as appropriate. In contrast, when the MRI system controller 22 gives displays of FIGS. 9A to 9C and accepts a given selected graph from those graphs, the MRI system controller 22 may display LV slice images (e.g. of one row)) corresponding to the accepted graph. As described above, display of LV slice images or graphs is not limited to the above-described example, and it can be changed arbitrarily, e.g. only a portion of images or graphs is displayed or a mechanism where they can be invoked when necessary using the mutual correspondence relationship is implemented.

### (Others: Bull's eye map)

FIGS. 10A to 10B illustrate new types of bull's eye map for better visualization and understanding of relationships between all or any of BBTI, signal intensity which is related to perfusion, and LV slice numbers constituting substantially contiguous slices within a 3D image of the LV. FIGS. 10A to 10B illustrate new perfusion visualizations using a variation of the well known bull's eye technique. Here, in FIG. 10A, for a given slice number, concentric circles illustrate AHA segments with BBTI values being illustrated along the radial direction. Each of the segments preferably is color-coded for average signal intensity (SI) or relative perfusion value. In this visualization, a trend of SI as a function of BBTI for each cardiac segment can be easily seen and understood. In FIG. 10B, for a given BBTI value, the concentric circles also illustrate AHA segments but now slice numbers are illustrated along the radial direction. Here each of the segments preferably is also color-coded to represent the average SI or perfusion value -- and now in this visualization, a trend of SI as a function of slice number at a given BBTI can be easily seen and understood.

In other words, for example, the MRI system controller creates, as an example of analysis results, a bull's eye map where the radial direction of concentric circles serves as the BBTI direction and displays the created bull's eye map. Furthermore, for example, the MRI system controller 22 creates a bull's eye map where the radial direction of concentric circles serves as the slice direction and displays the created bull's eye map. FIGS. 10A and 10B depict the AHA six segmentations as an example, but segmentation may be of other types, such as 17 segmentation. This also applies to the above-described other types of analysis.

The exemplary analysis methods are especially designed for use with inventors' non-contrast dynamic myocardial perfusion techniques. The whole analysis procedure helps to visualize perfusion of blood inside myocardium, and to distinguish infarcted regions or ischemic regions from healthy myocardium. The generated perfusion curves are important for quantified evaluation of ischemic disease or infarction. Color map image, perfusion curve graph, bull's eye map, etc. have been described as method of displaying analysis results. However, the MRI system controller 22 may display those that are necessary, for example, according to the request from the operator.

### (Exemplary computer program code structure)

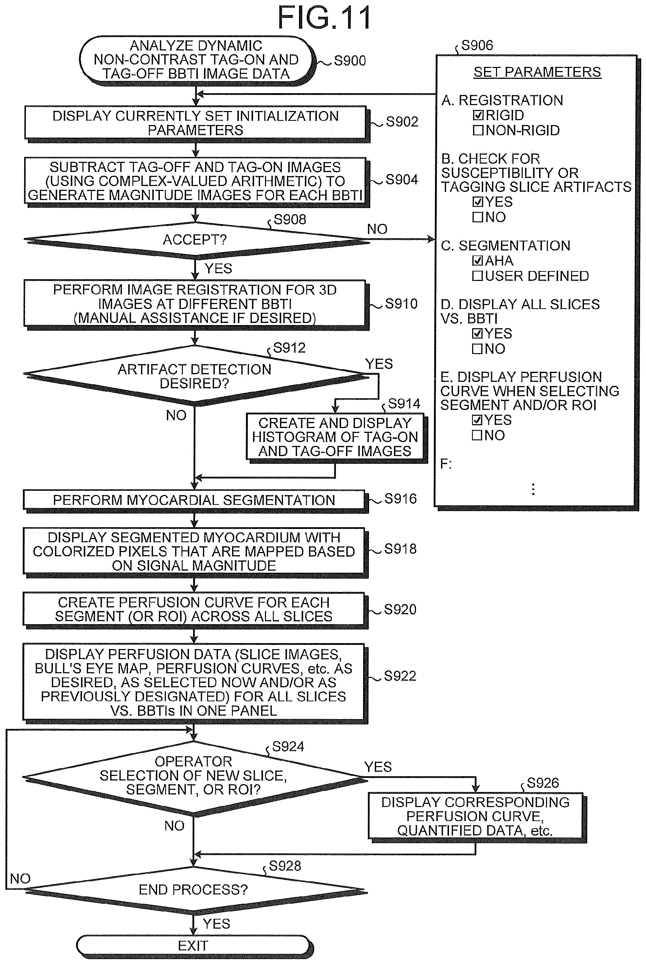

FIG. 11 is a schematic illustration of an exemplary computer program code structure for use in the MRI sysem of FIG. 1 (or a separate image processing apparatus) in the form of a flow chart for implementing exemplary embodiments of a system for analyzing BBTI tag-on and tag-off images acquired by magnetic resonance imaging. Step S900 in FIG. 11 depicts entry into an analysis of dynamic non-contrast tag-on and tag-off BBTI image data. As those in the art will appreciate, this flow chart represents executable computer program code structures such as those found in a computer program subroutine that can be called by a higher level application program or operating system.

If desired, the MRI system controller 22 displays the current (initially or last used) set initialization parameters (step S902) and generates intensity images at respective BBTIs (step S904). The MRI system controller 22 confirms whether the operator accepts these initialization parameters (step S908) and, if the operator does not accept them (NO at step S908), then displays the parameter initialization screen shown at S906 so that further operator adjustments/inputs can be accepted. These parameters are displayed again at step S902 and acceptance or rejection of the parameters is determined at step S908. For such parameters, some choices are prepared, such as, A. which of rigid registration and non-rigid registration is performed, B. whether to check there is an artifact, C. which of AHA segmentation and user-defined segmentation is performed, D. whether to perform BBTI one-panel display for all slides, E. whether to display perfusion curves. In the example of FIG. 11, parameters are set after the processing at step S904, but the processes are not necessarily performed in this order. For example, only the processes at steps S902, S908, S906 may be performed without step S904.

Once the initialization setting of the subroutine is found acceptable (if indeed the operator is even given an opportunity for such adjustments), then the MRI system controller 22 perform subtraction between previously acquired tag-on and tag-off images using complex-valued arithmetic to generate intensity images for each BBTI value and for each slice of a 3D image (step S904).

Subsequently, the MRI system controller 22 performs image registration for 3D images at different BBTI values (step S910). If desired, manual assistance may be permitted to effect image registration. Of course, image registration is not necessarily required.

If artifact detection is desired (YES at step S912), then the MRI system controller 22 creates histograms of tag-on and tag-off images and displays the created histograms. Subtraction is performed so as to provide data representing susceptibility errors and/or errors caused by a tagging pulse affecting the myocardium during image data acquisition. As those in the art will appreciate, the MRI system controller 22 can detect whether error above a certain threshold is present and, if there is error, perform compensation and/or request operator assistance or perhaps even terminate the process.

The MRI system controller 22 may perform myocardial segmentation, if desired (step S916). The MRI system controller 22 then displays images of segmented myocardium with pixels color-mapped according to signal intensity (step S918). The MRI system controller 22 creates perfusion curves for each segment (or ROI) for each slice of the 3D image. The MRI system controller 22 may display LV slice images and/or perfusion curves with respect to BBTI values (step S922). As previously discussed, the colorized slice images for the 3D image are preferably displayed in one panel as a function of BBTI values. Similarly, the perfusion curves for each segment and slice of the 3D image are preferably displayed in a single panel.

If the LV slices are displayed in a single panel, then an operator is given an opportunity to select a particular segment or ROI at the display (e.g., with a mouse or by touch or the like) (step S924). If such an operator selection is made, then the MRI system controller 22 then displays the corresponding perfusion curve for that particular segment (step S926). The operator is given an option for ending the processes (step S928). When ending the processes is desired, then the MRI system controller 22 ends this subroutine and control is passed back to the calling higher level program or operating system.

Displaying multi-slice images of any type (i.e., not just perfusion-related images but also non-contrast magnetic resonance images such as from magnetic resonance angiography (MRA) along a BBTI axis is believed to be new and advantageous. For example, a computerized system for analyzing images acquired by magnetic resonance imaging may include at least one computer processor coupled to associated memory, display and input/output ports and be configured to: (a) acquire multi-slice non-contrast MR images of left ventricle myocardium for each of multiple BBTI intervals in a region of interest (ROI); and (b) display apical to basal LV slice images as a function of BBTI for multiple slices of a 3D image and for multiple BBTI values in a single display panel. Such visualization of MR slices as a function of BBTI in a single display panel (e.g., similar to visualizations shown in all or any of FIGS. 7A, 7B, 9A to 9C, and 10A to 10B) will help operators to more quickly "see" important relationships between a succession of BBTI values and various types of MR images acquired with varying BBTI values.

### (Other embodiments)

Embodiments are not limited to the above-described embodiments.

### (Specific numerical values, Procedure)

The specific numerical values and procedure exemplified in the above-described embodiments are examples only. For example, an example of procedure is described using FIG. 11 for the above-described embodiment but embodiments are not limited to the procedure shown in FIG. 11 and it can be changed as required according to the mode of operation.

### (Trigger Signal)

In the above-described embodiment, an example has been described where data is acquired using an ECG signal as a trigger signal for ECG synchronization, but embodiments are not limited to this. Instead of ECG signal, other biosignals, such as pulse wave and respiratory signals, or clock signals of the MRI system, etc. may be used.

### (Image Processing Apparatus)

For the above-described embodiment, an example has been described where an MRI system performs all of data acquisition, analysis, and display of analysis results, but embodiments are not limited to this. For example, an image processing system including an MRI system and an image processing apparatus may perform above-described various processes. Here, the image processing apparatus is, for example, an image store device (image server) or a viewer of a work station or a PACS (Picture Archiving and Communication System), or various devices of an electronic health record system. In this case, the MRI system acquire data using the MRI sequence controller 30. In contrast, the image processing apparatus includes a controller equivalent to the MRI system controller 22 and receives MR data or k-space data acquired by the MRI system via a network from MRI apparatus or an image server, or accepts such data via inputs made by the operator via a recording medium, and stores the data in the memory. The image processing apparatus may perform the above-described various processes (e.g. the analysis process or display control process performed by the MRI system controller 22) on the MR data and k-space data stored in the memory.

As described above, according to a magnetic resonance imaging apparatus and an image processing apparatus according to at least one embodiment, BBTI (Black Blood Time to Inversion) tag-on and tag-off images acquired by magnetic resonance imaging (MRI) are analyzed to generate 3D images with different intensities as a function of the time (BBTI value) representing blood perfusion in a region of interest (ROI). For example, perfusion data for the ROI with different values for normal and abnormal myocardial tissues for multiple slices of a 3D image and multiple BBTI values is displayed in one display panel.
(1) A computerized system for analyzing images acquired by magnetic resonance imaging, the system including: at least one computer processor coupled to associated memory, display and input/output ports and configured to: (a) acquire multi-slice non-contrast MR images of left ventricle (LV) myocardium for each BBTI in a region of interest (ROI); and (b) display apical to basal LV slice images for multiple slices of a 3D image and for multiple BBTI values in a single display panel.
(2) A computerized system for analyzing BBTI tag-on and tag-off images acquired by magnetic resonance imaging, the system comprising: at least one computer processor coupled to associated memory, display and input/output ports and configured to: (a) subtract previously acquired tag-off and tag-on images on a pixel-by-pixel basis using complex-valued arithmetic for each of multiple BBTIs to generate difference intensity 3D images as a function of time representing blood perfusion in a region of interest (ROI); and (b) display perfusion data of the ROI having values which are different for normal and abnormal myocardial tissues for multiple slices of a 3D image and for multiple BBTI values in a single display panel.
(3) The system of (2), wherein the at least one computer processor is further configured to effect 3D image registration between the 3D images.
(4) The system of (3), wherein the at least one computer processor is further configured to perform rigid or non-rigid 3D image registration.
(5) The system of (4), wherein the at least one computer processor is further configured to accept manual operator alignment inputs to effect the 3D image registration.
(6) The system of (2), wherein the at least one computer processor is further configured to (a) effect segmentation of imaged left ventricle myocardial tissue in the ROI; and (b) display LV slice images as a function of BBTI in one display panel wherein the displayed LV slice images include at least one of: (i) LV slice images prior to segmentation; (ii) LV slice images after segmentation; and (iii) LV slice images after AHA (American Heart Association) segmentation.
(7) The system of (2) wherein the ROI includes left ventricle myocardium and the at least one computer processor is further configured to display the perfusion data in at least one bull's eye configuration showing at least one of: (a) a given segmented slice of LV myocardium having perfusion values plotted along concentric circles for each of different BBTI values plotted along a radial direction; and (b), for a given BBTI value, LV myocardial perfusion values plotted along concentric circles for each of different slices of the LV myocardium plotted along a radial direction.
(8) The system of (2), wherein the at least one computer processor is further configured (a) to generate histograms and (b) to subtract the histograms for tag-on and tag-off images with the subtraction results providing data representing susceptibility artifact and/or artifact caused by a tagging pulse affecting the myocardium images during image data acquisition processes.
(9) The system of (2), wherein the at least one computer processor is further configured to (a) perform coronary artery territory segmentation for left ventricle myocardium image data in the ROI, (b) generate an average image data value for each segmented portion of the left ventricle myocardium, and (c) display the segmented left ventricle myocardium with segmented portions being displayed with visual differences representing their respectively associated different average image data values.
(10) The system of (2), wherein the at least one computer processor is further configured to (a) generate at least one blood perfusion curve as a function of BBTI for each slice of a 3D image, and (b) simultaneously display the resulting multiple perfusion curves in one display panel showing at least one of: (a) an array of raw perfusion data as a function of BBTI before curve fitting, (b) an array of perfusion curves fitted to raw perfusion data as a function of BBTI, and (c) quantified perfusion curve data obtained from perfusion curves fitted to raw perfusion data as a function of BBTI.
(11) The system of (2), wherein the at least one computer processor is further configured to generate a blood perfusion curve as a function of BBTI for each of multiple segmented portions of the ROI for each slice of a 3D image and to simultaneously display a respectively corresponding perfusion curve when a segment of a slice is operator selected.
(12) A magnetic resonance imaging system including: an MRI gantry having a static magnet, a gradient coil, and at least one RF (Radio Frequency) coil defining an image volume into which a patient region of interest (ROI) can be disposed; MRI control circuits connected to control components within the MRI gantry and configured to effect MRI data acquisition sequences of RF and gradient magnetic pulses which elicit MRI signals from patient tissue when an ROI is disposed in the gantry, to acquire and process MR image data in conjunction (a) with use of an initial spatially-selective RF tag pulse (tag-on) in a data acquisition subsequence and (b) without use of an initial spatially-selective RF tag pulse (tag-off) in a data acquisition subsequence; the MRI control circuits being configured to (a) acquire multi-dimensional MR k-space data for a patient ROI using the tag-on and tag-off data acquisition subsequences for each of multiple time to inversion (TI) intervals without injecting a contrast agent; (b) reconstruct the acquired k-space data into spatial domain tag-on and tag-off MR image data; (c) subtract the acquired tag-on and tag-off MR image data for each of multiple TI intervals using complex-valued arithmetic to generate difference intensity image data as a function of time representing blood perfusion in the ROI having a corresponding flow time and/or relative peak blood flow intensity, or lack thereof, for the ROI which differentiates between normal, ischemic and infarct tissues; and (d) display perfusion data of the ROI having values which are different for normal, ischemic and infarct tissues for multiple slices of a 3D image and for multiple BBTI values in a single display panel.

The magnetic resonance imaging apparatus according to at least one of the above-described embodiments allows appropriate display of results of analysis of non-contrast myocardial perfusion images.

While certain embodiments have been described, these embodiments have been presented by way of example only, and are not intended to limit the scope of the inventions.

## Claims

1. A magnetic resonance imaging apparatus comprising:
a sequence controller (30) configured to perform, for each of multiple black blood time to inversion, hereinafter referred to as BBTI, of different lengths, data acquisition for acquiring, at a same cardiac phase, data on slices of a heart after each of the multiple BBTIs since application of a tag pulse on a tag region; and
a display controller (20) configured to display analysis images that are multiple results of analysis of the data acquired for the multiple BBTIs, in one panel and in a matrix whos axes represent a direction of the multiple BBTIs and a direction of the slices such that a relationship between the multiple BBTIs and the slices can be identified, each of the analysis images being a color map image in which a color corresponding to signal values in a segment is allocated on a segment-by-segment basis.

2. The magnetic resonance imaging apparatus according to claim 1, wherein
the sequence controller (30) is configured to acquire data on multiple apical to basal slices as the data on the slices of the heart.

3. The magnetic resonance imaging apparatus according to claim 1, wherein the display controller (20) is configured to display, as the analysis images, in the one panel and in the matrix, images in which a color of different densities is allocated depending on signal intensity of each segment, or images in which different colors are allocated depending on signal intensity of each segment.

4. The magnetic resonance imaging apparatus according to claim 1, wherein the display controller (20) is configured to display, as the multiple results of the analysis of the data, a graph of perfusion curves, wherein the graph is created by analyzing the data on the slices on a segment-by -segment basis and the perfusion curves represent changes of signal intensity in a direction of the given times.

5. The magnetic resonance imaging apparatus according to claim 4, wherein, upon accepting from an operator a selection operation regarding images displayed, the display controller (20) is configured to display the graph of the perfusion curves corresponding to an image selected by the operator.

6. The magnetic resonance imaging apparatus according to claim 1, wherein the display controller (20) is configured to display, as the multiple results of the analysis of the data, a bull's eye map in which the data on the slices is analyzed on a segment-by-segment basis, in which different densities or different colors are allocated depending on signal intensity of each segment and which is created with a radial direction of concentric circles serving as a direction of the multiple given times.

## Patentansprüche

1. Magnetresonanz-Bildgebungsvorrichtung, die Folgendes umfasst:
eine Sequenzsteuerung (30), die dafür konfiguriert ist, für jede von mehreren Schwarzblut-Inversionszeiten (black blood time to inversion), im Folgenden als BBTI bezeichnet, unterschiedlicher Längen eine Datenerfassung durchzuführen, zum, bei einer gleichen Herzphase, Erfassen von Daten über Schichten eines Herzes nach jeder der mehreren BBTI seit der Anwendung eines Markierungsimpulses auf einen Markierungsbereich, und
eine Anzeigesteuerung (20), die dafür konfiguriert ist, Analysebilder, die mehrere Ergebnisse der Analyse der für die mehreren BBTI erfassten Daten sind, in einem Feld und in einer Matrix anzuzeigen, deren Achsen eine Richtung der mehreren BBTI und eine Richtung der Schichten anzeigen so dass eine Beziehung zwischen den mehreren BBTI und den Schichten identifiziert werden kann, wobei jedes der Analysebilder ein Farbkartenbild ist, in dem eine Farbe, die Signalwerten in einem Segment entspricht, Segment für Segment zugewiesen wird.

2. Magnetresonanz-Bildgebungsvorrichtung nach Anspruch 1, wobei
die Sequenzsteuerung (30) dafür konfiguriert ist, Daten über mehrere apikale bis basale Schichten als die Daten über die Schichten des Herzes zu erfassen.

3. Magnetresonanz-Bildgebungsvorrichtung nach Anspruch 1, wobei die Anzeigesteuerung (20) dafür konfiguriert ist, als die Analysebilder, in dem einen Feld und in der Matrix, Bilder, in denen eine Farbe mit unterschiedlichen Dichten in Abhängigkeit von der Signalintensität jedes Segments zugewiesen wird, oder Bildern in denen unterschiedliche Farben in Abhängigkeit von der Signalintensität jedes Segments zugewiesen werden, anzuzeigen.

4. Magnetresonanz-Bildgebungsvorrichtung nach Anspruch 1, wobei die Anzeigesteuerung (20) dafür konfiguriert ist, als die mehreren Ergebnisse der Analyse der Daten eine graphische Darstellung von Perfusionskurven anzuzeigen, wobei die graphische Darstellung durch Analysieren der Daten über die Schichten Segment für Segment erzeugt wird und die Perfusionskurven Änderungen der Signalintensität in einer Richtung der gegebenen Zeiten darstellen.

5. Magnetresonanz-Bildgebungsvorrichtung nach Anspruch 4, wobei, auf das Annehmen einer Auswahloperation bezüglich angezeigter Bilder von einem Bediener hin, die Anzeigesteuerung (20) dafür konfiguriert ist, die graphische Darstellung der Perfusionskurven entsprechend einem durch den Bediener ausgewählten Bild anzuzeigen.

6. Magnetresonanz-Bildgebungsvorrichtung nach Anspruch 1, wobei die Anzeigesteuerung (20) dafür konfiguriert ist, als die mehreren Ergebnisse der Analyse der Daten eine Polarkarte anzuzeigen, in der die Daten über die Schichten Segment für Segment analysiert werden, in der unterschiedliche Dichten oder unterschiedliche Farben in Abhängigkeit von der Signalintensität jedes Segments zugewiesen werden und erzeugt wird, wobei eine radiale Richtung konzentrischer Kreise als eine Richtung der mehreren gegebenen Zeiten dient.

## Revendications

1. Appareil d'imagerie par résonance magnétique comprenant :
un contrôleur de séquence (30) qui est configuré de manière à ce qu'il réalise, pour chacun de multiples temps de sang noir jusqu'à inversion, ci-après appelés BBTI, de différentes longueurs, une acquisition de données pour acquérir, pour une même phase cardiaque, des données concernant des tranches d'un coeur après chacun des multiples BBTI depuis l'application d'une impulsion de marquage sur une région de marquage ; et
un contrôleur d'affichage (20) qui est configuré de manière à ce qu'il affiche des images d'analyse qui sont de multiples résultats d'analyse des données qui sont acquises pour les multiples BBTI, dans un panneau et dans une matrice dont les axes représentent une direction des multiples BBTI et une direction des tranches de telle sorte qu'une relation entre les multiples BBTI et les tranches puisse être identifiée, chacune des images d'analyse étant une image de carte en couleur dans laquelle une couleur qui correspond à des valeurs de signal dans un segment est attribuée sur une base segment par segment.

2. Appareil d'imagerie par résonance magnétique selon la revendication 1, dans lequel :
le contrôleur de séquence (30) est configuré de manière à ce qu'il acquière des données concernant de multiples tranches apicales à basales en tant que données concernant des tranches du coeur.

3. Appareil d'imagerie par résonance magnétique selon la revendication 1, dans lequel le contrôleur d'affichage (20) est configuré de manière à ce qu'il affiche, en tant qu'images d'analyse, dans le panneau considéré et dans la matrice, des images dans lesquelles une couleur de différentes densités est attribuée en fonction d'une intensité de signal de chaque segment, ou des images dans lesquelles différentes couleurs sont attribuées en fonction de l'intensité de signal de chaque segment.

4. Appareil d'imagerie par résonance magnétique selon la revendication 1, dans lequel le contrôleur d'affichage (20) est configuré de manière à ce qu'il affiche, en tant que multiples résultats de l'analyse des données, un graphique de courbes de perfusion, dans lequel le graphique est créé en analysant les données concernant les tranches sur une base segment par segment et les courbes de perfusion représentent des modifications de l'intensité de signal dans une direction des temps donnés.

5. Appareil d'imagerie par résonance magnétique selon la revendication 4, dans lequel, suite à l'acceptation, en provenance d'un opérateur, d'une opération de sélection concernant des images qui sont affichées, le contrôleur d'affichage (20) est configuré de manière à ce qu'il affiche le graphique des courbes de perfusion qui correspond à une image qui est sélectionnée par l'opérateur.

6. Appareil d'imagerie par résonance magnétique selon la revendication 1, dans lequel le contrôleur d'affichage (20) est configuré de manière à ce qu'il affiche, en tant que multiples résultats de l'analyse des données, une carte pleine cible dans laquelle les données concernant les tranches sont analysées sur une base segment par segment, dans laquelle différentes densités ou différentes couleurs sont attribuées en fonction de l'intensité de signal de chaque segment et qui est créée avec une direction radiale de cercles concentriques qui joue le rôle de direction des multiples temps donnés.
